# EUROPEAN PATENT SPECIFICATION

(11) **EP 2 195 330 B1**
(45) Date of publication and mention of the grant of the patent: **13.03.2013**
(21) Application number: 08802397.3
(22) Date of filing: 19.09.2008
(51) Int. Cl.: C07D 213/74, C07K 1/113, C07D 265/30

(54) **Method and agent for refolding proteins**
Verfahren und Mittel zur Faltung von Proteinen
Procédé et agent pour le repliement de protéines

(30) Priority: 12.10.2007 US 979542 P
(43) Date of publication of application: 16.06.2010
(73) Proprietor: Merck Patent GmbH, 64293 Darmstadt (DE)
(72) Inventor: PITNER, William-Robert, 60599 Frankfurt (DE); EICHHORN, Jens, 64354 Reinheim (DE); VON HAGEN, Joerg, 64319 Pfungstadt (DE); LELAND, Peter, A., Madison WI 53711 (US); SCOTT, Graham, B., I., Trinity TX 75862 (US)
(86) International application number: PCT/EP2008/007886
(87) International publication number: WO 2009/046840

(56) References cited:
- EP-A- 1 734 047
- WO-A-03/051908
- CHRISTIAN LANGE, GANESH PATIL, RAINER RUDOLPH: "Ionic liquids as refolding additives: N'-alkyl and N'-(omega-hydroxyalkyl) N-methylimidazolium chlorides" PROTEIN SCIENCE, vol. 14, no. 10, October 2005 (2005-10), pages 2693-2701, XP002502253
- SCOTT SEARLES, VANCE P. GREGORY: "The Reaction of Trimethylene Oxide with Amines" JOURNAL OF THE AMERICAN CHEMICAL SOCIETY, vol. 76, 20 May 1954 (1954-05-20), pages 2789-2790, XP002502254
- JERCHEL D ET AL: "SYNTHESEN MIT PYRIDYL-PYRIDINIUM-HALOGENIDEN: EINFUEHRUNG VON HALOGEN, DER THIOL- UND THIOAETHERGRUPPE IN DIE 4-STELLUNG DES PYRIDINKERNS" CHEMISCHE BERICHTE, VERLAG CHEMIE GMBH. WEINHEIM, DE, vol. 89, no. 12, 1 January 1956 (1956-01-01), pages 2921-2933, XP001194471 ISSN: 0009-2940
- LALL SHARON ET AL: "Polycations-12. The synthesis of liquid ionic phosphates (LIPs) from mono- and polycationic ammonium halides" SYNTHESIS, GEORG THIEME VERLAG, STUTTGART, DE, no. 11, 1 January 2002 (2002-01-01), pages 1530-1540, XP002466819 ISSN: 0039-7881
- TAN ET AL: "Purification and refolding optimization of recombinant bovine enterokinase light chain overexpressed in Escherichia coli" PROTEIN EXPRESSION AND PURIFICATION, ACADEMIC PRESS, SAN DIEGO, CA, vol. 56, no. 1, 3 October 2007 (2007-10-03), pages 40-47, XP022284711 ISSN: 1046-5928

## Description

This invention relates to the use of ionic liquids comprising a cation with at least one electron donor region and one positively charged electrostatic region which are spatially distinct from each other for protein refolding and/or protein stabilization and methods for refolding and/or stabilizing proteins using said ionic liquids.

### Background of the invention

One important process in biotechnology is the expression of recombinant proteins. Due to the developments achieved in molecular biology it is possible to clone proteins starting with the coding sequences thereof and to produce them in host organisms in a recombinant manner. Upon high-yield production of recombinant proteins in bacteria, the recombinant proteins often precipitate in the form of biologically inactive aggregates or inclusion bodies. The proteins contained in these inclusion bodies must subsequently be transformed into the biologically active form by means of in vitro refolding.

In a first step, the inclusion bodies are solubilized typically with denaturing agents or chaotropic agents like urea or guanidinium hydrochloride. That means the recombinant proteins are denatured (completely unfolded) and solubilized (aggregates are dissolved). In most circumstances, it is necessary to include a reducing agent in the denaturation and solubilization step. The reducing agent acts to break non-native inter- and intra-chain disulfide bonds.

In a second step, after denaturation and solubilization, the proteins must be refolded into the native and active three dimensional conformation. Renaturation or refolding methods are designed to transfer the target protein from a denaturing environment into an environment that favours the correctly folded, active protein. For the vast majority of target proteins, the refolding environment is critical. If it is incorrect, the target protein fails to refold, re-aggregates and precipitates again.

Many different molecules that facilitate protein refolding have been identified, e.g. salts, buffers, glycols, amino acids or sugars. It is for example known that the addition of molecules such as urea or guanidinium in a non-denaturing concentration can have a positive impact on the efficiency of refolding. As an alternative to guanidinium or urea other chaotropic substances have been used in in vitro folding processes, for example alkyl urea, or organic co-solvents such as carboxylic acid amides or alkylated amines.

It has also been observed that the yield of in vitro folding can be enhanced by the addition of L-arginine. Other additives which increase the efficiency of in vitro folding are highly concentrated Tris buffers, polyethylene glycol or detergents.

WO 03/051908 discloses the use of special ionic liquids, i.e. substituted imidazolium salts, as refolding agents.

Christian Lange et al., Protein Science, Vol. 14, No. 10, October 2010 (2005-10), pages 2693-2701 disclose the use of hydroxyalkyl substituted methylimidazolium chlorides as additives for refolding recombinant proteins.

Despite this large collection of refolding additives, there are many recombinant proteins that remain intractable to refolding or are refolded in bad yields.

Consequently, there exists a clear need for novel, highly efficacious protein refolding additives.

### Brief description of the Invention

It has been found that ionic liquids with a certain distribution of electron density are especially efficient protein refolding and stabilizing agents. The said ionic liquids need to comprise a cation with at least one electron donor region and at least one positively charged electrostatic region which are spatially distinct from each other.

The present invention therefore relates to the use of ionic liquids comprising a cation with at least one electron donor region and at least one positively charged electrostatic region which are spatially distinct from each other for the refolding, for the increase of the thermal stability and/or for the decrease of aggregation of proteins.
The ionic liquids comprising a cation with at least one electron donor region and at least one positively charged electrostatic region which are spatially distinct have a cation with one of the following general structures A or B

[HetN]⁺-ED A

or

[HetNED]⁺ B,

with
HetN being an aromatic, partially aromatic or non-aromatic heterocyclic ring system with at least one nitrogen atom being part of the ring system.

ED being an electron donor. According to structure A, ED is a substituent which is covalently bound to one atom of the heterocyclic ring system, but ED is not part of the ring system.
According to structure B, ED is part of the heterocyclic ring system, that means ED is an electron donor group which is directly incorporated in the ring structure.

The cation with at least one electron donor region and at least one positively charged electrostatic region which are spatially distinct from each other is selected from the group 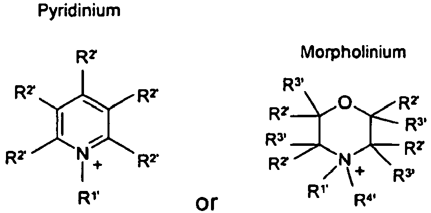 whereby Morpholinium is an example for [HetNED]⁺ because the oxygen atom in the ring can serve as an electron donor, and the other structure given is an example for [HetN]⁺-ED where the electron donor function is provided by at least one of the substituents R^{1'} to R^{4'},
and where the substituents R^{1'}to R^{4'} each, independently of one another, denote
-H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂, -NO₂,
straight-chain or branched alkyl having 1-20 C atoms,
straight-chain or branched alkenyl having 2-20 C atoms and one or more double bonds,
straight-chain or branched alkinyl having 2-20 C atoms and one or more triple bonds,
saturated, partially or fully unsaturated cycloalkyl having 3-7 C atoms, which may be substituted by alkyl groups having 1-6 C atoms, saturated, partially or fully unsaturated heteroaryl, heteroaryl-C₁-C₆-alkyl or aryl-C₁-C₆-alkyl,
where the substituents R^{1'}, R^{2'} , R^{3'} and/or R^{4'} together may also form a ring system,
where one or more substituents R^{1'} to R^{4'} may be partially or fully substituted by halogens, in particular -F and/or -Cl, or -OH, -OR', -CN, -C(O)OH, -C(O)NR'₂, -SO₂NR'₂, -C(O)X, -SO₂OH, -SO₂X or -NO₂, but where R^{1'} and R^{4'} cannot simultaneously be fully substituted by halogens, and where one or two non-adjacent carbon atoms of the substituents R^{1'} to R^{4'} which are not bonded to the heteroatom may be replaced by atoms and/or atom groups selected from -O-, -S-, -S(O)-, -SO₂-, -SO₂O -C(O)-, -C(O)O-, -N⁺R'₂-, -P(O)R'O-, -C(O)NR'-, -SO₂NR'- -OP(O)R'O-, -P(O)(NR'₂)NR'-, -PR'₂=N- and -P(O)R'-, where R' = H, non-, partially or perfluorinated C₁- to C₆-alkyl, C₃- to C₇-cycloalkyl, or unsubstituted or substituted phenyl, and
X = halogen,
with the proviso that the Pyridinium cation has at least one substituent R^{1'} to R^{4'} per molecule which is -CN, -OR', -NR'₂, -P(O)R'₂, -P(O)(OR')₂, -P(O)(NR'₂)₂, -C(O)R', -C(O)OR', -C(O)NR'₂, -SO₂NR' or -NO₂
and/or at least one substituent R^{1'} to R^{4'} per molecule which is substituted with -OR', -CN, -C(O)OH, -C(O)NR'₂, -SO₂NR'₂, -C(O)X, -SO₂OH -SO₂X or -NO₂
and/or at least one substituent R^{1'} to R^{4'} per molecule in which one or two non-adjacent carbon atoms which are not bonded to the heteroatom are replaced by atoms and/or atom groups selected from -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- and -P(O)R'- .
That means that if there is no electron donor function already integrated in the ring structure as in Morpholinium at least one substituent R^{1'} to R^{4'} needs to comprise one of the said electron donor features.

In a preferred embodiment, the cation of the ionic liquid according to the present invention comprises only one electron donor region.

For the purposes of the present invention, fully unsaturated substituents are also taken to mean aromatic substituents.

The substituents R' are particularly preferably methyl, ethyl, isopropyl, propyl, n-butyl, sec-butyl, tert-butyl, pentyl or hexyl.

In a preferred embodiment, one but only one substituent R^{1'} to R^{4'} per molecule is -CN, -OR', -NR'₂, -P(O)R'₂, -P(O)(OR')₂, -P(O)(NR'₂)₂, -C(O)R', -C(O)OR', -C(O)NR'₂, -SO₂NR' or -NO₂.

In a preferred embodiment, one but only one substituent R^{1'} to R^{4'} per molecule is -CN, -OR', -NR'₂, -P(O)R'₂, -P(O)(OR')₂, -P(O)(NR'₂)₂, -C(O)R', -C(O)OR', -C(O)NR'₂, -SO₂NR'₂ or -NO₂ and the other substituents R^{1'} to R^{4'} independently of one another, denote -H or straight-chain or branched alkyl having 1-20 C atoms, preferably -H, methyl, ethyl, isopropyl, propyl, n-butyl, sec-butyl, tert-butyl, pentyl or hexyl.

The most preferred morpholinium cations are 4-(cyanomethyl)-4-methylmorpholinium and 4-(3-hydroxyethyl)-4-methylmorpholinium.

In a very preferred embodiment, the cation with at least one electron donor region and at least one positively charged electrostatic region which are spatially distinct from each other is selected from the group 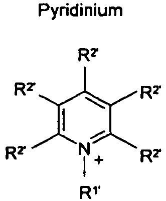 with
one but only one R^{2'} being selected from the group dimethylamino or diethylamino, Most preferred is dimethylamino
and the other R^{2'} being independently from another selected from the group -H, methyl, ethyl, iso-propyl, n-propyl, butyl, pentyl, hexyl. Most preferred is -H.
and R^{1'} being selected from the group methyl, ethyl, (iso-)propyl , n-propyl or n- butyl.

In a very preferred embodiment, the cation is N-methyl-4-(N',N'-dimethylamino)-pyridinium, N-ethyl-4-(N',N'-dimethylamino)-pyridinium, N-butyl-4-(N',N'-dimethylamino)-pyridinium.

In another preferred embodiment, the anion of the ionic liquid is Cl⁻, Br⁻, I⁻ or BF₄⁻.

The present invention is also directed to a method for the refolding, the increase of the thermal stability and/or the decrease of the aggregation of proteins, wherein the proteins to be treated are contacted with a liquid medium comprising at least one ionic liquid which comprises a cation with at least one electron donor region and at least one positively charged electrostatic region which are spatially distinct from each other selected from the group 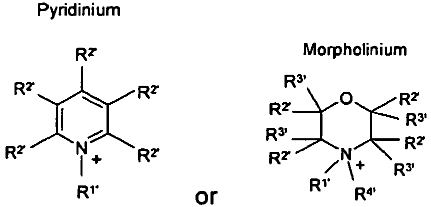 as defined above.

The preferred embodiments described above for the use according to the present invention are also the preferred embodiments used for the method for the refolding, the increase of the thermal stability and/or the decrease of the aggregation of proteins.

The present invention is further directed to a method for extracting proteins from inclusion bodies by
a) solubilizing the proteins from the inclusion bodies with denaturing agents or chaotropic agents like urea or guanidinium hydrochloride. That means the recombinant proteins are denatured (completely unfolded) and solubilized (aggregates are dissolved).
b) refolding the proteins solubilized in step a) by contacting them with a liquid medium comprising at least one ionic liquid which comprises a cation with at least one electron donor region and at least one positively charged electrostatic region which are spatially distinct from each other selected from the group 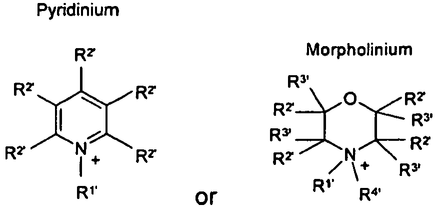 as defined above.

Further disclosed is an agent comprising at least one ionic liquid which comprises a cation with at least one electron donor region and at least one positively charged electrostatic region which are spatially distinct from each other in a liquid medium.

In a preferred embodiment, the agent additionally comprises one or more of the following substances:
- reduction agents like *tris*(2-Carboxyethyl)phosphine, reduced glutathione, 1,4-Dithiothreitol, or 2-Mercaptoethanol
- a redox system like a mixture of oxidized and reduced glutathione or a mixture of cysteine and cystine
- other substances that are known to promote refolding of proteins like urea, guanidinium, L-arginine, alkyl urea, carboxylic acid amides, alkylated amines, Tris buffers, polyethylene glycol, detergents, sugars, and/or zwitterionic molecules

The most preferred ionic liquids for use according to the present invention are the chlorides, bromides and/or iodides of N-methyl-4-(N',N'-dimethylamino)-pyridinium, N-ethyl-4-(N',N'-dimethylamino)-pyridinium, N-butyl-4-(N',N'-dimethylamino)-pyridinium, 4-(cyanomethyl)-4-methylmorpholinium and/or 4-(3-hydroxyethyl)-4-methylmorpholinium, for example 4-(3-hydroxyethyl)-4-methylmorpholiniumiodide, 4-(3-hydroxypropyl)-4-methylmorpholinium chloride, N-ethyl-4-(N,N-dimethylamino)pyridinium bromide or *N*-methyl-4-(*N,N-*dimethylamino)pyridinium iodide.

The preferred embodiments described above for the use according to the present invention are also the preferred embodiments used for the method for the refolding, the increase of the thermal stability and/or the decrease of the aggregation of proteins and for the method for extracting proteins form inclusion bodies.

### Description of the drawings

Figure 1 shows results from refolding MMP12 when ionic liquids are included in the refolding buffer. Further details can be found in Example 2.
Figure 2 shows results from refolding trx-GFP when ionic liquids are included in the refolding buffer. Further details can be found in Example 3.
Figure 3 shows results from refolding EK when the ionic liquid *N*-ethyl-4-(*N,N*-dimethylamino)pyridinium bromide is included in the refolding buffer.
Further details can be found in Example 4.
Figure 4 shows results from refolding A PPase when the ionic liquid *N*-methyl-4-(*N,N*-dimethylamino)pyridinium iodide is included in the refolding buffer at varied concentrations. Further details can be found in Example 5.
Figure 5 shows the stability of bovine hemoglobin at elevated temperature in the presence of the ionic liquid *N*-ethyl-4-(*N,N*-dimethylamino) pyridinium bromide. Further details can be found in Example 6.
Figure 6 comprises Figure 6A and Figure &A and shows results from refolding the scFv-trx fusion protein in presence of the ionic liquid 4-(3-hydroxyethyl)-4-methylmorpholinium iodide in comparison to non-detergent sulfobetaine (Figure 6A) and trehalose and polyethylene glycol (Figure 6B).

Abbreviations used in figures, tables and elsewhere have the following meanings:
BCA means Bicinchoninic Acid
bis-TRIS means 2,2-Bis(hydroxymethyl)-2,2',2"-nitrilotriethanol
BME means 2-Mercaptoethanol
bmim Cl means 1-butyl-3-methylimidazolium chloride
Brij-35 means C₁₂E₂₃ Polyoxyethylene(23) lauryl ether
BSA means bovine serum albumin
CHES means 2-(Cyclohexylamino)ethanesulfonic acid
DTE means *erythro-*1,4-Dimercapto-2,3-butanediol
DTT means 1,4-Dithiothreitol
EDTA means Ethylenediaminetetraacetic acid
EK means the catalytic subunit of bovine enterokinase
emim Cl means 1-ethyl-3-methylimidazolium chloride
EPPS means 4-(2-Hydroxyethyl)-1-piperazinepropanesulfonic acid
Hepes means 4-(2-Hydroxyethyl)piperazine-1-ethanesulfonic acid
λ PPase means lambda protein phosphatase
Mes means 2-(N-Morpholino)ethanesulfonic acid
MMP12 means the catalytic subunit from human matrix metalloprotease 12
Mops means 3-(N-Morpholino)propanesulfonic acid
TAPS means [(2-Hydroxy-1,1-bis(hydroxymethyl)ethyl)amino]-1-propanesulfonic acid
TCEP means tris(2-carboxyethyl)phosphine
THP means Tris(hydroxypropyl)phosphine
Tris means Tris(hydroxymethyl)aminomethane
trx-GFP means a fusion protein comprised of the bacterial thioredoxin protein and the green fluorescent protein

### Description of the invention

Ionic liquids or liquid salts are ionic species which consist of an organic cation and an inorganic or (less frequently) an organic anion. They do not comprise neutral molecules. That means the ionic liquids used according to the invention are liquid at room temperature, or if they are not liquid at room temperature, they should at least be present in a liquid form and/or be soluble in the liquid medium under the conditions of treatment. For example, N-ethyl-4-(N',N'-dimethylamino)-pyridinium is solid at room temperature but highly soluble (>2.0 M) in biologically relevant buffers (pH 5.0 - 9.0) at temperatures from 4-37°C.

Intensive research is currently being carried out in the area of ionic liquids since the potential applications are multifarious. Review articles on ionic liquids are, for example, R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionic Flüssigkeiten - neue Lösungen für die Übergangsmetallkatalyse" [Ionic Liquids - Novel Solutions for Transition-Metal Catalysis], Angew. Chem., 112 (2000), 3926-3945; T. Welton "Room temperature ionic liquids. Solvents for synthesis and catalysis", Chem. Rev., 92 (1999), 2071-2083 or R. Hagiwara, Ya. Ito "Room temperature ionic liquids of alkylimidazolium cations and fluoroanions", J. Fluorine Chem., 105 (2000), 221-227.

According to the present invention, "ionic liquids comprising a cation with at least one electron donor region and at least one positively charged electrostatic region which are spatially distinct from each other" preferably means that the ionic liquid comprises a cation with at least one electron donor region which is spatially distinct from the positively charged electrostatic region of the cation.

It is possible to have a cation with more than one positively charged electrostatic region (a divalent cation, or dication) which are spatially distinct from each other.
For example, 1,4-methyllpiperazine can be converted to the divalent 1,1,4,4-tetramethylpiperazinium cation, but in a preferred embodiment, the ionic liquids of the present invention only comprise a cation with one positively charged electrostatic region.

"spatially distinct" means that there are typically at least two bonds between the electron donor region and the positively charged electrostatic region. In a preferred embodiment, there are at least two bonds between the centre of the electron donor region and the centre of the positively charged electrostatic region. Centre means the group of atoms or the one atom at which the electrons of the electron donor region are mainly focused or the group of atoms or the atom at which the positive charge is mainly focused.

According to the invention, an electron donor region is a region containing a an electron releasing group. An electron releasing group is a functional group which is able to release electrons into a reaction center.
Examples of electron releasing groups are alcohol groups, cyano groups, ether groups and amino groups.

Examples of cations with electron donor regions are N-alkyl-dimethylamminopyridinium and N,N-dialkylmorpholinium.

According to the present invention, a positively charged electrostatic region is a region with a positive electrostatic potential, due to a deficit of electrons.
This region can be a point charge, located on a single atom, or delocalized, where the charge is distributed over two or more atoms.
In a non-aromatic cation (such as morpholinium or pyrrolidinium), the positively charged electrostatic region is localised in the region immediately surrounding the ring-nitrogen atom, the ring-nitrogen atom being the centre. In an aromatic cation (such as pyridinium or imidazolium), the positive charge is delocalised and distributed (but not evenly) amongst the atoms of the ring.
Refolding according to the present invention means the renaturation of a protein, i.e. the regaining of the native state of the protein in which it shows its biological activity. Typically, the proteins which have to be refolded are denatured proteins. Refolding typically means a change of the 3-dimensional order of the protein.

According to the invention, the decrease of aggregation is intended to mean a decrease or even a substantial prevention of the aggregation of proteins which usually occurs during prolonged storage periods in liquid media and which is accompanied by a loss or a reduction in biological functionality.

According to the invention, increase of the thermal stability means that the biological activity or the correct protein folding, respectively, are maintained over a prolonged period of time even at temperatures which can be by far higher than room temperature.

According to the present invention a refolding agent, also called refolding medium or refolding liquid, is the liquid mixture with which the proteins to be refolded are contacted. It typically comprises at least one liquid medium and at least one type of ionic liquid comprising a cation with at least one electron donor region and at least one positively charged electrostatic region which are spatially distinct from each other. The refolding agent may comprise further additives like stabilizing agents, reduction agents like TCEP or glutathione, a redox system (e.g. composed of reduced and oxidized thiol substances such as DTT, DTE, glutathione, cysteine, mercaptoethanol) or other substances that are known to promote refolding like for example urea, guanidinium, L-arginine, alkyl urea, carboxylic acid amides, alkylated amines, Tris buffers, polyethylene glycol, detergents, sugars, and/or zwitterionic molecules (non-detergent sulfobetaines). The refolding agent according to the invention may also comprise two or more different ionic liquids comprising a cation with at least one electron donor region and at least one positively charged electrostatic region which are spatially distinct from each other.

Suitable examples of cations for ionic liquids comprising a cation with at least one electron donor region and at least one positively charged electrostatic region which are spatially distinct from each other are given above and in the claims.

Suitable anions are all anions that can be used to generate ionic liquids with the above mentioned cations with at least one electron donor region and at least one positively charged electrostatic region which are spatially distinct from each other. In a preferred embodiment, the anion of the ionic liquid is Cl⁻, Br⁻, I⁻ or BF₄⁻.

The agents and the method according to the present invention are suitable for the refolding of any protein or any class of proteins. The protein might be a protein which has been produced by chemical synthesis or which has been extracted from viruses, cells or tissues, e.g. from prokaryotic or eukaryotic cells. The agents and the method according to the present invention are especially suitable for the refolding of recombinant proteins. Typically, the recombinant proteins have been expressed in host cells like bacterial cells, e.g. *E. coli* cells. In this context, inclusion bodies are defined as dense, insoluble, mis-folded aggregates of recombinant protein, located in the cytoplasm and/or the periplasmic space of the host cell. In addition to the recombinant protein, inclusion bodies may contain host cell proteins, nucleic acids, lipids, and/or other host cell molecules.
Other suitable sources of protein to be refolded include, but are not limited to, recombinant proteins produced in yeast, insect, fungal, mammalian cells, or *in vitro* translation systems that do fold into the native three dimensional structure.

Inclusion bodies may be isolated from the host cell using any one of the many well-established protocols.

Examples of proteins which can be refolded according to the method of the present invention are:
Proteases, preferably serine proteases, particularly thrombin, factor Xa, caspases, cathepsins, trypsin and chymotrypsin, cysteine proteases, acidic proteases such as pepsin or rennin, metalloproteinases such as thermolysin; protease inhibitors, preferably pepstatins, antipain, chemostatins, elastinal, leupeptins, bestatin, antithrombin III;
DNA binding proteins, preferably transcription factors, particularly NF kappa B and members of the jun, fos, krox, myc, E2F families, viral T antigens;
viral proteins, e.g. viral envelope proteins, capsid proteins, viral proteases, polymerases and/or T antigens;
phosphatases;
protein kinases, preferably tyrosine kinases and/or serine kinases;
proteins of the immunoglobulin superfamily, e.g. antibodies and fragments thereof; growth factors, e.g. epidermal growth factor (EGF), erythropoietin, fibroblast growth factor (FGF), insulin-like growth factors I and II (IGF I and IGF II), interleukin-2 (IL-2), nerve growth factor (NGF), transforming growth factor beta. (TGF-.beta.) and/or thrombocyte growth factor (PDGF),
as well as proteins and fragments derived from these proteins.

The agents and the method according to the present invention are especially suitable for the refolding of:
Bovine enterokinase (EK for *Enter*oKnase). Protein Data Bank (PDB) identification number 1 EKB.

Matrix Metalloprotease 12 (MMP12). PDB identification number 2OXU.

Fusion protein of thioredoxin and green fluorescent protein (trx-GFP). Trx PDB identification number 2TRX. GFP PDB identification number 1 B9C.

Lambda protein phosphatase (λ PPase). PBD identification number 1G5B.

Human rhino virus protease 3C (HRV 3C). PDB identification number 1 CQQ.

According to the invention, disulfide-free and disulfide-bridged proteins can be refolded. Multidomain proteins and complex disulfide-bridged proteins can be treated such as e.g. lysozyme, enterokinase, rPA (recombinant plasminogen activator, trade name replase), alpha glucosidase, antibodies and fragments derived therefrom and/or growth factors. These proteins/protein classes merely serve as examples.

As the liquid medium in the method according to the invention for the refolding of proteins there is preferably used an aqueous medium, i.e. water, an aqueous buffer system or mixtures of water or aqueous buffer systems with water soluble organic solvents like ethanol, butyl alcohol, acetonitrile, etc. (typically not more than 20Vol% organic solvent.) Suitable buffers are Tris, Hepes, Mes, Mops, EPPS, TAPS, CHES, bis-TRIS, acetate, glycine and/or phosphate. The buffer concentration in the liquid medium is preferably between 10 and 1000 mM, more preferably between 5 and 200 mM, also preferred between 10 and 200 mM and further preferred between 10 and 50 mM. The pH of the liquid medium preferably is between 4-11, further preferred between 6.5-9.0. For most proteins good results can be achieved with a pH between 7.0 and 8.5. In the case of recombinant enterokinase, for example, the pH value during renaturation is preferably about 7.0-8.5, and for the renaturation of the thioredoxin-green fluorescent protein, the pH value preferably is about 6.5-8.5. For other proteins to be refolded the buffer composition parameters may be adapted and optimized individually to obtain a maximal yield of refolded protein.

It is preferable, but not necessary, to reduce the amount of contaminating substances in the preparation of protein to be refolded prior to refolding. Contaminating substances may be reduced or removed by washing the protein preparation, typically the inclusion bodies comprising the protein to be refolded, with a buffered aqueous solution containing a wash agent. Suitable wash agents include, but are not limited to, mild detergents, detergent-like molecules, chaotropes, and salts. Alternatively, contaminating substances may be reduced or removed by first denaturing and reducing the recombinant protein (described below) and applying the resulting preparation to a chromatography resin. Typically, the recombinant protein is selectively immobilized on the chromatography resin and contaminating substances do not bind to the resin or are washed away with a buffered aqueous solution containing a chaotropic agent. The recombinant protein is eluted from the chromatography resin by adjusting the buffer conditions such that the strength of the interaction between the target protein and the resin is reduced significantly. Wash protocols can be adjusted by those skilled in the art with respect to the protein to be refolded and the nature of the contaminating substances.

Prior to refolding, it is often necessary to fully solubilize and denature the target protein. A fully solubilized protein should be primarily monomeric and lack tertiary structure. Preferably, solubilization is accomplished by contacting the mis-folded, aggregated, and/or insoluble target protein with a buffered aqueous solution containing a high concentration of a chaotropic agent, such as guanidine hydrochloride, urea, or N-lauryl sarcosine and a reducing agent such as DTT, BME, THP, or TCEP.

In the method according to the invention the contacting of the proteins in a solubilized and denatured state to be refolded or to be treated is preferably performed by diluting, dialyzing and/or diafiltrating the protein to be treated with the refolding liquid. Principally, a buffer exchange of the denatured protein into the refolding liquid should be assured for the refolding.

During contacting with the refolding agent and particularly during the refolding, the protein concentration of the protein to be treated preferably is 5-500 µg/ml, preferably 10-200 µg/ml, still more preferably about 100-200 µg/ml. These values can be adjusted by those skilled in the art with respect to the protein to be refolded or to be treated, respectively, considering the solubility properties of the respective protein.

The method according to the invention is useful for the refolding of disulfide-free and disulfide-bridged proteins. Regarding disulfide-free proteins, these are preferably contacted with a refolding medium that further comprises a reduction agent such as DTT, DTE, glutathione and/or cysteine, preferably in a concentration of 1-10 mM.

Regarding the refolding of disulfide-bridged proteins, these are preferably contacted in the presence of a redox system composed of reduced and oxidized thiol substances such as DTT, DTE, glutathione, cysteine, mercaptoethanol, preferably in a concentration of 1-10 mM. In these cases, the concentration ratios of reduced substances to oxidized substances ("reduced:oxidized") preferably are 1:10 to 20:1, preferably 1:5-10:1, further preferred 1:1 to 5:1.

During contacting and particularly during the refolding the concentration of the ionic liquids comprising a cation with at least one electron donor region and at least one positively charged electrostatic region which are spatially distinct from each other typically is approximately 0.25-5 M, preferably 0.3 to 1.5 M, most preferred 0.5 to 1.0 M.

Generally, the period of refolding preferably is: 0.1-100 h, further preferred 1-50 h, still more preferably about 2-24 h.

The refolding preferably takes place at low temperatures of 0-37 °C, preferably 5-20°C, since at higher temperatures the aggregation reactions increase.

Typically, as a parameter for the refolding efficiency, the biological activity of the protein and the aggregation behaviour can be measured over the course of the process or afterwards.

In another embodiment of the method according to the invention the protein may be added to the refolding agent at several successive times in a pulsed manner or in a continuous manner. Preferably, the refolding procedure is done batch wise, that means, the whole amount of protein is added to a suitable amount of the refolding agent at one time.

Depending on the down-stream application it might be necessary to remove the refolding agent, especially the ionic liquid comprised in the refolding agent from the refolded proteins. Some of the ionic liquids can interfere with assays for biological or enzymatic activity. In these circumstances, the concentration of the ionic liquid is reduced (essentially to zero) by using dialysis. Proteins are retained within the dialysis membrane and the IL equilibrates with the dialysis buffer.

Refolding efficiency varies greatly between target proteins and the quality of the inclusion body preparation (amount and type of contaminants).

Without further elaboration, it is believed that one skilled in the art can, using the preceding description, utilise the present invention to its fullest extent.

### Examples

The following examples represent practical applications of the invention.

### EXAMPLE 1

Denaturation (or solubilization) and reduction of proteins expressed as inclusion bodies.

Proteins in the form of inclusion bodies are denatured, solubilized, and reduced by stirring inclusion bodies in a solution of 50 mM TRIS, pH 8.0, 7.0 M guanidine hydrochloride, 0.2 M NaCl, 2.0 mM EDTA, and 10 mM TCEP for approximately 2 hours at room temperature. The sample is centrifuged at 25,000 x g for 15 minutes at 4°C and then passed through a 0.45 µm filter to remove any insoluble material. The concentration of the protein sample is determined using the bicinchoninic acid (BCA) method (for *example, see:* Smith, P.K., et al. (1985). Anal. Biochem. 150, 76-85 or *product number 71285 from EMD Chemicals or product number* 23225 *from ThermoFisher) .*

### EXAMPLE 2

Refolding of matrix metalloprotease 12 using N-ethyl-4-(N,N-dimethylamino)pyridinium bromide, 1-butyl-3-methylimidazolium chloride, or 1-ethyl-3-methylimidazolium chloride.

The refolding of matrix metalloprotease 12 (MMP12), present in 50 mM TRIS, pH 8.0, 7.0 M guanidine hydrochloride, 0.2 M NaCl, 2.0 mM EDTA and 10 mM TCEP, is performed by rapidly diluting the protein into the refolding buffer at a ratio of 1:50. The final concentration of the protein is 100 µg/mL. The refolding buffer is 50 mM bis-TRIS, pH 6.5, containing 0.5 M of the ionic liquid *N*-ethyl-4-(*N,N*-dimethylamino)pyridinium bromide, 1-butyl-3-methylimidazolium chloride, or 1-ethyl-3-methylimidazolium chloride. Refolding samples are incubated at 22 ± 2°C for 20-24 hours with shaking at 300 RPM. After refolding, samples are dialyzed against 50 mM TRIS, pH 7.5, 0.15 M NaCl, 2.0 mM CaCl₂, 1.0 µM ZnCl₂ and 0.03% (v/v) Brij-35 at a sample to buffer ratio of 1:40,000, or greater, for 20-24 hours at 10 ± 2°C. Refolding is measured using an enzymatic activity assay specific for MMP12. The substrate used to measure MMP12 enzymatic activity is BODIPY-FL-labeled DQ elastin conjugate, available from Invitrogen (catalog number E12056).

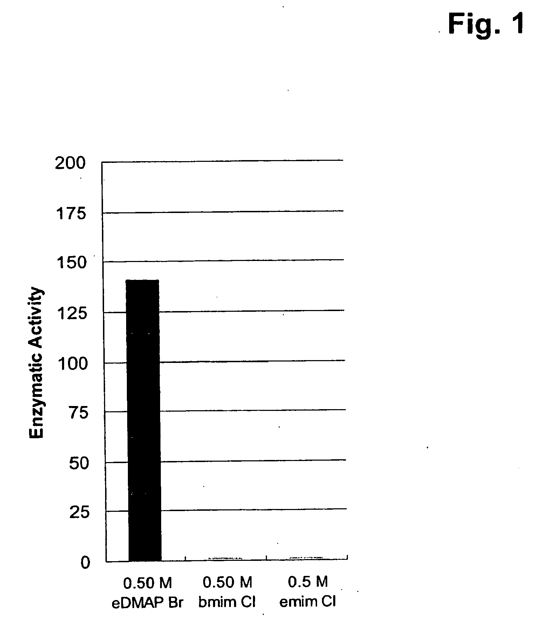

Figure 1 shows results from refolding MMP12 when ionic liquids are included in the refolding buffer. Refolding of MMP12 is far more efficient in a 0.5 M solution of the pyridium-based ionic liquid *N*-ethyl-4-(*N,N-*dimethylamino)pyridinium bromide than is refolding of MMP12 in a 0.5 M solution of the imidazolium-based ionic liquids 1-butyl-3-methylimidazolium chloride (bmim Cl) or 1-ethyl-3-methylimidazolium chloride (emim Cl).

### EXAMPLE 3

Refolding of the thioredoxin-green fluorescent protein fusion using 4-(3-hydroxypropyl)-4-methylmorpholinium chloride and comparison to the refolding agents L-arginine, NaCl, and non-detergent sulfobetaine 256.

The refolding of the thioredoxin-green fluorescent protein fusion (trx-GFP), present in 50 mM TRIS, pH 8.0, 7.0 M guanidine hydrochloride, 0.2 M NaCl, 2.0 mM EDTA and 10 mM TCEP, is performed by rapidly diluting the protein into the refolding buffer at a ratio of 1:50. The final concentration of the protein is 100 µg/mL. The refolding buffer is 50 mM TAPS, pH 8.5, 7.6 mM reduced glutathione, 2.4 mM oxidized glutathione and 0.5 M of the ionic liquid 4-(3-hydroxypropyl)-4-methylmorpholinium chloride or one of three control refolding additives: 0.5 M L-arginine, 0.25 M NaCl, or 1.0 M non-detergent sulfobetaine 256. Refolding samples are incubated at 22 ± 2°C for 20-24 hours with shaking at 300 RPM. After refolding, samples are dialyzed against 50 mM TRIS, pH 8.0 at a sample to buffer ratio of 1:40,000, or greater, for 20-24 hours at 10 ± 2°C. The degree of refolding of trx-GFP fusion protein is judged by measuring the relative fluorescence intensity of the refolded samples, using an excitation wavelength of 388 nm and an emission wavelength of 504 nm.

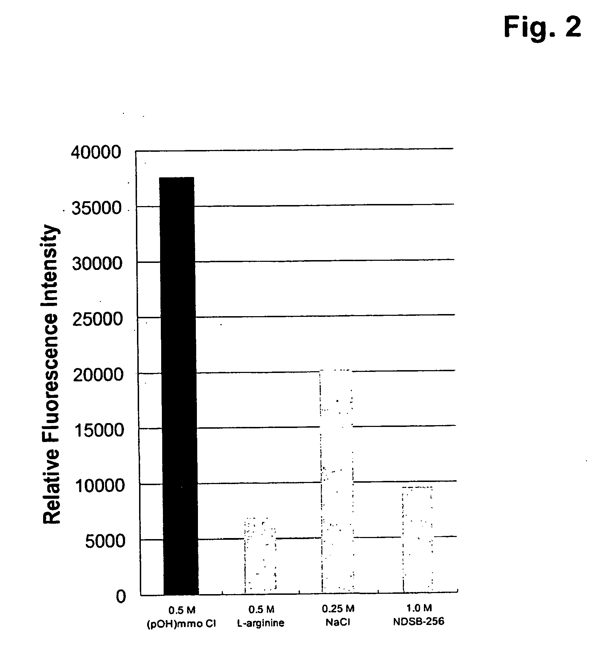

Figure 2 shows results from refolding trx-GFP when ionic liquids are included in the refolding buffer. Refolding of trx-GFP is far more efficient in a 0.5 M solution of the morpholinium-based ionic liquid 4-(3-hydroxypropyl)-4-methylmorpholinium chloride than is refolding trx-GFP in any of the three control protein refolding additives: 0.5 M L-arginine, 0.25 M NaCl, or 1.0 M non-detergent sulfobetaine.

### EXAMPLE 4

Refolding bovine enterokinase using *N*-ethyl-4-(*N,N-*dimethylamino)pyridinium bromide and comparison to the refolding agents L-arginine, sodium chloride, and non-detergent sulfobetaine 256.

The refolding of bovine enterokinase (EK), present in 50 mM TRIS, pH 8.0, 7.0 M guanidine hydrochloride, 0.2 M NaCl, 2.0 mM EDTA and 10 mM TCEP, is performed by rapidly diluting the protein into the refolding buffer at a ratio of 1:50. The final concentration of the protein is 100 µg/mL. The refolding buffer is 50 mM HEPES, pH 7.5, containing 1.0 M of the ionic liquid *N*-ethyl-4-(*N,N*-dimethylamino)pyridinium bromide or one of the three control refolding additives: 0.5 M L-arginine, 0.25 M NaCl, or 1.0 M non-detergent sulfobetaine 256. Refolding samples are incubated at 22 ± 2°C for 20-24 hours with shaking at 300 RPM. After refolding, samples are dialyzed against 20 mM TRIS, pH 7.5, 150 mM NaCl and 2.0 mM CaCl₂ at a sample to buffer ratio of 1:40,000 or greater for 20-24 hours at 10 ± 2°C.

refolding is measured by using an enzymatic activity specific for EK. The substrate used to measure enzymatic activity is a fluorescently-labeled peptide substrate, available from Sigma Aldrich (Gly-Asp-Asp-Asp-Asp-Lys-β-naphthylamide, catalog number G5261).

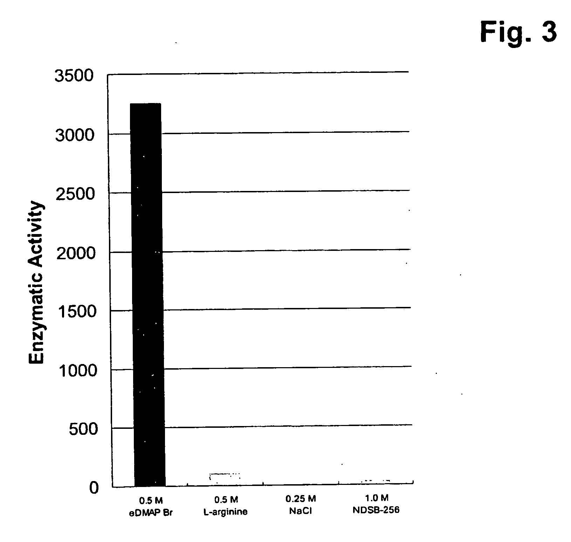

Figure 3 shows results from refolding EK when the ionic liquid *N*-ethyl-4-(*N,N*-dimethylamino)pyridinium bromide is included in the refolding buffer. Refolding of bovine enterokinase is far more efficient in a 1.0 M solution of the pyridinium-based ionic liquid *N*-ethyl-4-(*N*,*N*-dimethylamino)pyridinium bromide than is refolding of EK in any of the three control refolding additives: 0.5 M L-arginine, 0.25 M NaCl, or 1.0 M non-detergent sulfobetaine 256.

### EXAMPLE 5

Refolding lambda protein phosphatase using *N*-methyl-4-(*N*,*N-*dimethylamino)pyridinium iodide.

The refolding of lambda protein phosphatase (λ PPase), present in 50 mM TRIS, pH 8.0, 7.0 M guanidine hydrochloride, 0.2 M NaCl, 2.0 mM EDTA and 10 mM TCEP, is performed by rapidly diluting the protein into the refolding buffer at a ratio of 1:50. The final concentration of the protein is 200 µg/mL. The refolding buffer is 50 mM HEPES, pH 7.5, containing 7.6 mM reduced glutathione, 2.4 mM oxidized glutathione and varied concentrations of the ionic liquid *N*-methyl-4-(*N*,*N*-dimethylamino)pyridinium iodide. The refolding process is performed at 22 ± 2°C for 20-24 hours with shaking at 300 RPM. After refolding, samples are diluted 1:50 into 50 mM TRIS, pH 7.5, containing 5.0 mM DTT, 2 mM MgCl₂, 0.1 mM EDTA, 0.1% (w/v) BSA, and 0.01 % (v/v) Brij-35. Refolding is measured by using an enzymatic activity specific for λ PPase. The substrate used to measure enzymatic activity is 4-nitrophenyl phosphate, available from Sigma Aldrich (catalog number N22002).

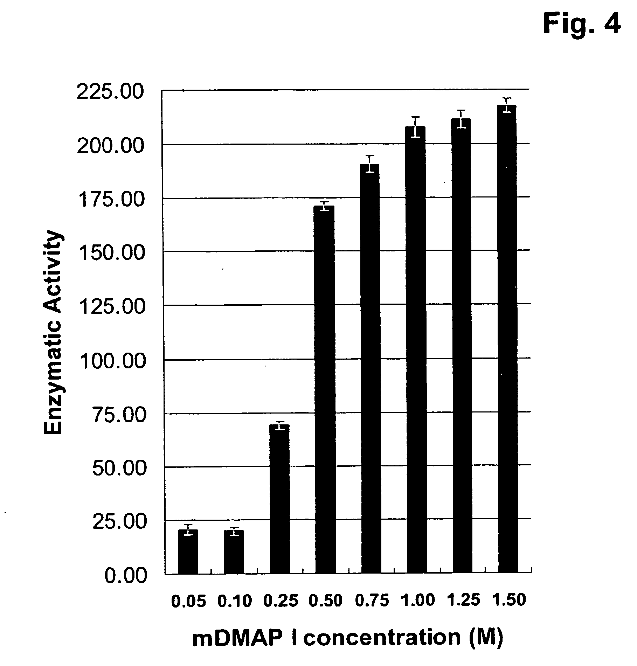

Figure 4 shows results from refolding λ PPase when the ionic liquid *N*-methyl-4-(*N*,*N*-dimethylamino)pyridinium iodide is included in the refolding buffer at varied concentrations. Refolding efficiency reaches a maximum when *N*-methyl-4-(*N,N*-dimethylamino)pyridinium iodide is present at 1.0 M.

### EXAMPLE 6

Stabilization of bovine hemoglobin at elevated temperature by the ionic liquid *N*-methyl-4-(*N,N*-dimethylamino)pyridinium chloride.

Native bovine hemoglobin (Sigma/Aldrich catalog number H2625) is dissolved in 0.1 M sodium phosphate, pH 7.4, 0.4 M NaCl) at a final concentration of 10.0 mg/mL. Aliquots of this hemoglobin solution are diluted two-fold with 1.5 M *N*-methyl-4-(*N,N*-dimethylamino) pyridinium chloride, 100% glycerol, distilled and deionized H₂O, or 1.5 M NaCl to make the following four test solutions:
(1) 50 mM sodium phosphate, pH 7.4, containing 0.2 M NaCl, 5.0 mg/mL hemoglobin, and 0.75 M *N*-methyl-4-(*N,N*-dimethylamino) pyridinium chloride (▲);
(2) 50 mM sodium phosphate, pH 7.4, containing 0.2 M NaCl, 5.0 mg/mL hemoglobin, and 50% (v/v) glycerol (■)
(3) 50 mM sodium phosphate, pH 7.4, containing 0.2 M NaCl, and 5.0 mg/mL hemoglobin (• control);
(4) 50 mM sodium phosphate, pH 7.4, containing 0.95 M NaCl, and 5.0 mg/mL hemoglobin (▼; 0.2 M NaCl from the base buffer and 0.75 M NaCl as an experimental trial).

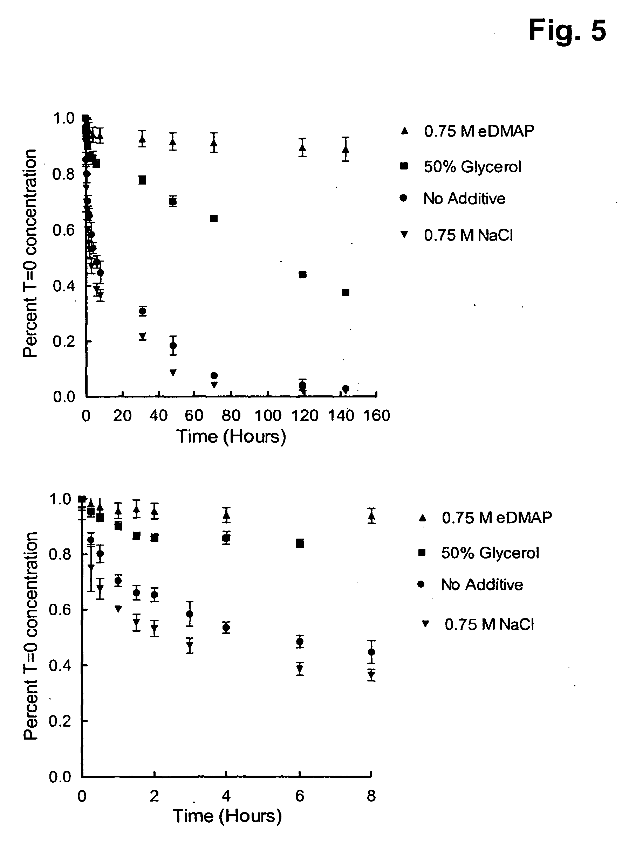

Each hemoglobin solution is incubated at 55°C for 143 hours. During the incubation, samples are removed from each hemoglobin solution and spun at 16,000xg for 15 min at room temperature to remove any precipitated haemoglobin. The amount of hemoglobin remaining in the soluble fraction is quantified by measuring the absorbance of the solution at 540 nm (the absorbance of hemoglobin at 540 nm changes linearly with the protein concentration). The data shown in figure 5 represents the average of three independent measurements for each condition at each time point. The standard deviation of each data point is shown. The upper figure shows data from the entire time course. The lower figure shows the same data for the first eight hours only.

Figure 5 shows that addition of *N*-methyl-4-(*N,N*-dimethylamino)pyridinium chloride to a final concentration of 0.75 M (▲) significantly delays, or, prevents the precipitation of a 5.0 mg/mL solution of bovine hemoglobin in 50 mM sodium phosphate, pH 7.4, 0.2 M NaCl incubated at 55°C. The stabilizing effect of 0.75 M *N*-methyl-4-(*N,N*-dimethylamino)pyridinium chloride (▲) is far greater than that of a 50% (v/v) solution of glycerol, which is a traditional protein stabilization agent. The stabilizing effect of 0.75 M *N*-methyl-4-(*N,N*-dimethylamino)pyridinium chloride (▲) is not due to an increase in the ionic strength of the solution as the addition of 0.75 M NaCl to the base buffer (▼, 0.95 M total NaCl) accelerates precipitation of the hemoglobin compared to the control sample (•).

### EXAMPLE 7

Refolding of a single-chain antibody thioredoxin fusion protein using 4-(3-hydroxyethyl)-4-methylmorpholinium iodide and comparison to the refolding agents non-detergent sulfobetaine 256, trehalose, and polyethylene glycol 3350.

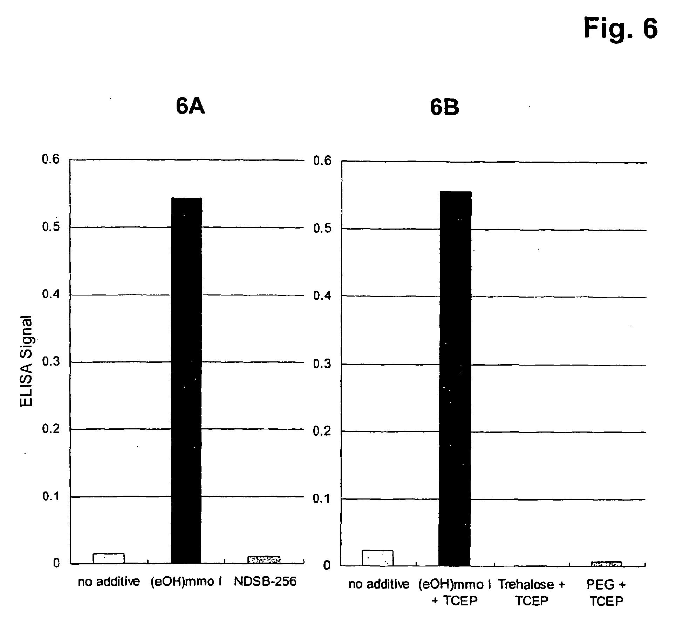

Refolding of the single-chain antibody thioredoxin fusion protein (scFv-trx), present in 50 mM TRIS, pH 8.0, 7.0 M guanidine hydrochloride, 0.2 M NaCl, 2.0 mM EDTA and 10 mM TCEP, is performed by rapidly diluting the protein into the refolding buffer at a ratio of 1:50 for refolding buffers containing 4-(3-hydroxyethyl)-4-methylmorpholinium iodide (final protein concentration of 200 µg/mL) or at a ratio of 1:100 for refolding buffers containing non-detergent sulfobetaine 256, trehalose, or polyethylene glycol 3350 (final protein concentration of 100 µg/mL). For Figure 6A, the refolding buffer is 50 mM EPPS, pH 8.0. For figure 6B the refolding buffer is 50 mM TAPS, pH 8.5, containing 1.0 mM TCEP. 4-(3-hydroxyethyl)-4-methylmorpholinium iodide is added to the refolding buffer to a final concentration of 1.0 M. Non-detergent sulfobetaine 256, trehalose, and polyethylene glycol 3350 are added to the refolding buffer to final concentrations of 1.0 M, 0.58 M, and 0.06% (w/v), respectively. Refolding samples are incubated at 22±2°C for 20-24 hours with shaking at 300 RPM. After refolding, samples are dialyzed against 25 mM HEPES, pH 7.5, containing 0.2 M NaCl, 0.5 mM DTT, 10% (v/v) glycerol, and 0.03% (v/v) BRIJ 35 at a sample to buffer ratio of 1:40,000 or greater for 20-24 hours at 10±2°C. The degree of refolding was measured using an ELISA specific for the correctly folded scFv-trx fusion protein.

Figure 6A shows results from refolding of the scFv-trx fusion protein when the ionic liquid 4-(3-hydroxyethyl)-4-methylmorpholinium iodide ((eOH)mmo I) is included in the 50 mM EPPS, pH 8.0, refolding buffer in comparison to refolding results in the absence of an additive or when non-detergent sulfobetaine (NDSB-256) is included in the refolding buffer. Refolding of the scFv-trx fusion protein is far more efficient in a 1.0 M solution of 4-(3-hydroxyethyl)-4-methylmorpholinium iodide than is refolding of the scFv-trx fusion protein in the absence of a refolding additive or in the presence of 1.0 M non-detergent sulfobetaine 256.

Figure 6B shows results from refolding of the scFv-trx fusion protein when the ionic liquid 4-(3-hydroxyethyl)-4-methylmorpholinium iodide ((eOH)mmo I) is included in the 50 mM TAPS, pH 8.0, 1.0 mM TCEP refolding buffer in comparison to refolding results in the absence of an additive or when 0.58 M trehalose or 0.06% (w/v) polyethylene glycol (PEG3350) is included in the refolding buffer. Refolding of the scFv-trx fusion protein is far more efficient in a 1.0 M solution of 4-(3-hydroxyethyl)-4-methylmorpholinium iodide than is refolding of the scFv-trx fusion protein in the absence of a refolding additive or in the presence of 0.58 M trehalose or 0.06% (w/v) polyethylene glycol.

## Claims

1. Use of ionic liquids comprising a cation with at least one electron donor region and at least one positively charged electrostatic region which are spatially distinct from each other and with one of the following general structures A or B
[HetN]⁺-ED A
or
[HetNED]⁺ B,
with
HetN being an aromatic, partially aromatic or non-aromatic heterocyclic ring system with at least one nitrogen atom being part of the ring system and
ED being an electron donor for the refolding, for the increase of the thermal stability and/or for the decrease of aggregation of proteins; **characterized in that** the cation with at least one electron donor region and at least one positively charged electrostatic region which are spatially distinct from each other is selected from the group 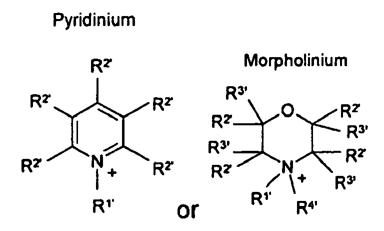 where R^{1'} to R^{4'} each, independently of one another, denote -H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂ -NO₂,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, saturated, partially or fully unsaturated heteroaryl, heteroaryl-C₁-C₆-alkyl or aryl-C₁-C₆-alkyl,
where the substituents R^{1'}, R^{2'} , R^{3'} and/or R^{4'} together may also form a ring system,
where one or more substituents R^{1'} to R^{4'} may be partially or fully substituted by halogens, in particular -F and/or -Cl, or -OH, -OR', -CN, -C(O)OH, -C(O)NR'₂, -SO₂NR'₂ -C(O)X, -SO₂OH, -SO₂X or -NO₂, but where R^{1'} and R^{4'} cannot simultaneously be fully substituted by halogens, and where one or two non-adjacent carbon atoms of the substituents R^{1'} to R^{4'} which are not bonded to the heteroatom may be replaced by atoms and/or atom groups selected from -O-, -S-, -S(O)-, -SO₂- -SO₂O-,-C(O)-, -C(O)O-, -N⁺R'₂-, -P(O)R'O-, -C(O)NR'-, -SO₂NR'- -OP(O)R'O-, -P(O)(NR'₂)NR'-, -PR'₂=N- and -P(O)R'-, where R' = H, non-, partially or perfluorinated C₁- to C₆-alkyl, C₃- to C₇-cycloalkyl, or unsubstituted or substituted phenyl, and X = halogen, with the proviso that the Pyridinium cation has at least one substituent R^{1'} to R^{4'} per molecule which is -CN, -OR', -NR'₂, -P(O)R'₂, -P(O)(OR')₂, -P(O)(NR'₂)₂, -C(O)R', -C(O)OR', -C(O)NR'₂, -SO₂NR'₂ or -NO₂ and/or at least one substituent R^{1'} to R^{4'} per molecule which is substituted with -OR', -CN, -C(O)OH, -C(O)NR'₂, -SO₂NR'₂, -C(O)X, -SO₂OH, -SO₂X or -NO₂ or in which one or two non-adjacent carbon atoms which are not bonded to the heteroatom are replaced by atoms and/or atom groups selected from -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- and -P(O)R'- .

2. Use according to claim 1, **characterized in that** substituents R' independently of one another are methyl, ethyl, isopropyl, propyl, butyl, sec-butyl, tert-butyl, pentyl or hexyl.

3. Use according to claims 1 and 2 **characterized in that** one but only one substituent R^{1'} to R^{4'} per molecule is -CN, -OR', -NR'₂, -P(O)R'₂, -P(O)(OR')₂, -P(O)(NR'₂)₂, -C(O)R', -C(O)OR', -C(O)NR'₂, -SO₂NR'₂ or - NO₂

4. Use according to one or more of claims 1 to 3 **characterized in that** one but only one substituent R^{1'} to R^{4'} per molecule is -CN, -OR', -NR'₂, -P(O)R'₂, -P(O)(OR')₂, -P(O)(NR'₂)₂, -C(O)R', -C(O)OR', -C(O)NR'₂, - SO₂NR'₂ or -NO₂ and the other substituents R^{1'} to R^{4'} independently of one another, denote -H or straight-chain or branched alkyl having 1-20 C atoms.

5. Use according to one or more of claims 1 to 4, **characterized in that** the cation is N-methyl-4-(N',N'-dimethylamino)-pyridinium, N-ethyl-4-(N',N'-dimethylamino)-pyridinium or N-butyl-4-(N',N'-dimethylamino)-pyridinium.

6. Use according to one or more of claims 1 to 5 **characterized in that** the anion of the ionic liquid is Cl⁻, BR⁻, I⁻ or BF₄⁻.

7. Method for the refolding, the increase of the thermal stability and/or the decrease of the aggregation of proteins, wherein the proteins to be treated are contacted with a liquid medium comprising at least one ionic liquid which comprises a cation with at least one electron donor region and at least one positively charged electrostatic region which are spatially distinct from each other and with one of the following general structures A or B
[HetN]⁺-ED A
or
[HetNED]⁺ B,
with
HetN being an aromatic, partially aromatic or non-aromatic heterocyclic ring system with at least one nitrogen atom being part of the ring system and
ED being an electron donor; **characterized in that** the cation with at least one electron donor region and at least one positively charged electrostatic region which are spatially distinct from each other is selected from the group 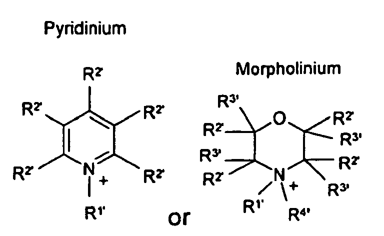 with R^{1'} to R⁴' each, independently of one another, denote -H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂, -NO₂,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
saturated, partially or fully unsaturated heteroaryl, heteroaryl-C₁-C₆-alkyl or aryl-C₁-C₆-alkyl,
where the substituents R^{1'}, R^{2'} , R^{3'} and/or R^{4'} together may also form a ring system,
where one or more substituents R^{1'} to R^{4'} may be partially or fully substituted by halogens, in particular -F and/or -Cl, or -OH, -OR', -CN, -C(O)OH, -C(O)NR'₂, -SO₂NR'₂, -C(O)X, -SO₂OH, -SO₂X or -NO₂, but where R^{1'} and R^{4'} cannot simultaneously be fully substituted by halogens, and where one or two non-adjacent carbon atoms of the substituents R^{1'} to R^{4'} which are not bonded to the heteroatom may be replaced by atoms and/or atom groups selected from -O-, -S-, -S(O)-, -SO₂-, -SO₂O-, -C(O)-, -C(O)O-, -N-R-₂-, -P(O)R'O-, -C(O)NR'-, -SO₂NR'- -OP(O)R'O-, -P(0)(NR'₂)NR'-. -PR'₂=N- and -P(O)R'-, where R' = H, non-, partially or perfluorinated C₁- to C₆-alkyl, C₃- to C₇-cycloalkyl, or unsubstituted or substituted phenyl, and X = halogen, with the proviso that the Pyridinium cation has at least one substituent R^{1'} to R^{4'} per molecule which is -CN, -OR', -NR'₂, -P(O)R'₂, -P(O)(OR')₂, -P(O)(NR'₂)₂, -C(O)R', -C(O)OR', -C(O)NR'₂, -SO₂NR'₂ or -NO₂ and/or at least one substituent R^{1'} to R^{4'} per molecule which is substituted with -OR', -CN, -C(O)OH, -C(O)NR'₂, -SO₂NR'₂, -C(O)X, -SO₂OH, -SO₂X or -NO₂ or in which one or two non-adjacent carbon atoms which are not bonded to the heteroatom are replaced by atoms and/or atom groups selected from -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- and -P(O)R'- .

8. Method for extracting proteins from inclusion bodies by
a) solubilizing the proteins from the inclusion bodies with denaturing agents or chaotropic agents,
b) refolding the proteins solubilized in step a) by contacting them with a liquid medium comprising at least one ionic liquid comprising a cation with at least one electron donor region and at least one positively charged electrostatic region which are spatially distinct from each other and with one of the following general structures A or B
[HetN]⁺-ED A
or
[HetNED]⁺ B,
with
HetN being an aromatic, partially aromatic or non-aromatic heterocyclic ring system with at least one nitrogen atom being part of the ring system and
ED being an electron donor; **characterized in that** the cation with at least one electron donor region and at least one positively charged electrostatic region which are spatially distinct from each other is selected from the group 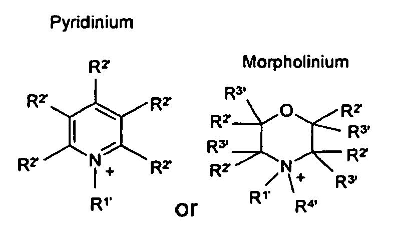 with R^{1'} to R^{4'} each, independently of one another, denote -H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂, -NO₂,
straight-chain or branched alkyl having 1-20 C atoms, straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, saturated, partially or fully unsaturated heteroaryl, heteroaryl-C₁-C₆-alkyl or aryl-C₁-C₆-alkyl,
where the substituents R^{1'}, R^{2'}, R^{3'} and/or R^{4'} together may also form a ring system,
where one or more substituents R^{1'} to R^{4'} may be partially or fully substituted by halogens, in particular -F and/or -Cl, or -OH, -OR', -CN, -C(O)OH, -C(O)NR'₂, -SO₂NR'₂, -C(O)X, -SO₂OH, -SO₂X or -NO₂, but where R^{1'} and R^{4'} cannot simultaneously be fully substituted by halogens, and where one or two non-adjacent carbon atoms of the substituents R^{1'} to R^{4'} which are not bonded to the heteroatom may be replaced by atoms and/or atom groups selected from -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- and -P(O)R'-, where R' = H, non-, partially or perfluorinated C₁- to C₆-alkyl, C₃- to C₇-cycloalkyl, or unsubstituted or substituted phenyl, and X = halogen, with the proviso that the Pyridinium cation has at least one substituent R^{1'} to R^{4'} per molecule which is -CN, -OR', -NR'₂, -P(O)R'₂, -P(O)(OR')₂, -P(O)(NR'₂)₂, -C(O)R', -C(O)OR', -C(O)NR'₂, -SO₂NR'₂ or -NO₂ and/or at least one substituent R^{1'} to R^{4'} per molecule which is substituted with -OR', -CN, -C(O)OH, -C(O)NR'₂, -SO₂NR'₂, -C(O)X, -SO₂OH, -SO₂X or -NO₂ or in which one or two non-adjacent carbon atoms which are not bonded to the heteroatom are replaced by atoms and/or atom groups selected from -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- and -P(O)R'- .

## Patentansprüche

1. Verwendung von ionischen Flüssigkeiten enthaltend ein Kation mit mindestens einer Elektronendonorregion und mindestens einer positiv geladenen elektrostatischen Region, die räumlich voneinander getrennt sind, und mit einer der folgenden allgemeinen Strukturen A oder B
[HetN]⁺-ED A
oder
[HetNED]⁺ B,
wobei
HetN ein aromatisches, teilaromatisches oder nichtaromatisches heterocyclisches Ringsystem mit mindestens einem Stickstoffatom, das Teil des Ringsystems ist, bedeutet und
ED einen Elektronendonor bedeutet für die Wiederfaltung, für die Erhöhung der thermischen Stabilität und/oder für die Verringerung der Proteinaggregation,
**dadurch gekennzeichnet, dass** das Kation mit mindestens einer Elektronendonorregion und mindestens einer positiv geladenen elektrostatischen Region, die räumlich voneinander getrennt sind, aus der Gruppe 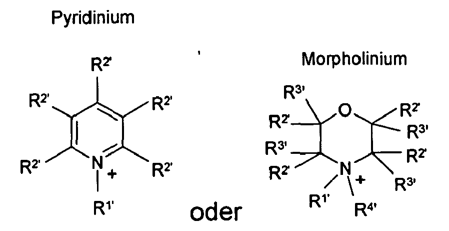 ausgewählt ist, wobei R^{1'} bis R^{4'} jeweils unabhängig voneinander bedeuten -H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂, -NO₂,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann, gesättigtes, teilweise oder vollständig ungesättigtes Heteroaryl, Heteroaryl-C₁-C₆-alkyl oder Aryl-C₁-C₆-alkyl,
wobei die Substituenten R^{1'}, R^{2'}, R^{3'} und/oder R^{4'} zusammen auch ein Ringsystem bilden können,
wobei ein oder mehrere Substituenten R^{1'} bis R^{4'} teilweise oder vollständig durch Halogene, insbesondere -F und/oder -Cl, oder -OH, -OR', -CN, -C(O)OH, -C(O)NR'₂, -SO₂NR'₂, -C(O)X, -SO₂OH, -SO₂X oder -NO₂, substituiert sein können, wobei aber R^{1'} und R^{4'} nicht gleichzeitig vollständig durch Halogene substituiert sein können, und wobei ein oder zwei nicht benachbarte Kohlenstoffatome der Substituenten R^{1'} bis R^{4'}, die nicht an das Heteroatom gebunden sind, ersetzt sein können durch Atome und/oder Atomgruppen ausgewählt aus -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- und -P(O)R'-, wobei R' = H, nicht, teilweise oder perfluoriertes C₁- bis C₆-Alkyl, C₃- bis C₇-Cycloalkyl oder unsubstituiertes oder substitutiertes Phenyl und X = Halogen,
mit der Maßgabe, dass das Pyridiniumkation mindestens einen Substituenten R^{1'} bis R^{4'} pro Molekül, bei dem es sich um -CN, -OR', -NR'₂, -P(O)R'₂, -P(O)(OR')₂, -P(O)(NR'₂)₂, -C(O)R', -C(O)OR', -C(O)NR'₂, -SO₂NR'₂ oder -NO₂ handelt, und/oder mindestens einen Substituenten R^{1'} bis R^{4'} pro Molekül, der mit -OR', -CN, -C(O)OH, -C(O)NR'₂, -SO₂NR'₂, -C(O)X, -SO₂OH, -SO₂X oder -NO₂ substituiert ist oder in dem ein oder zwei nicht benachbarte Kohlenstoffatome, die nicht an das Heteroatom gebunden sind, ersetzt sind durch Atome und/oder Atomgruppen ausgewählt aus -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- und -P(O)R'-, aufweist.

2. Verwendung nach Anspruch 1, **dadurch gekennzeichnet, dass** die Substituenten R' unabhängig voneinander Methyl, Ethyl, Isopropyl, Propyl, Butyl, sec.-Butyl, tert.-Butyl, Pentyl oder Hexyl bedeuten.

3. Verwendung nach den Ansprüchen 1 und 2, **dadurch gekennzeichnet, dass** ein und nur ein Substituent R^{1'} bis R^{4'} pro Molekül -CN, -OR', -NR'₂, -P(O)R'₂, -P(O)(OR')₂, -P(O)(NR'₂)₂, -C(O)R', -C(O)OR', -C(O)NR'₂, -SO₂NR'₂ oder -NO₂ bedeutet.

4. Verwendung nach einem oder mehreren der Ansprüche 1 bis 3, **dadurch gekennzeichnet, dass** ein und nur ein Substituent R^{1'} bis R^{4'} pro Molekül -CN, -OR', -NR'₂, -P(O)R'₂, -P(O)(OR')₂, -P(O)(NR'₂)₂, -C(O)R', -C(O)OR', -C(O)NR'₂, -SO₂NR'₂ oder -NO₂ bedeutet und die anderen Substituenten R^{1'} bis R^{4'} unabhängig voneinander -H oder geradkettiges oder verzweigtes Alkyl mit 1-20 C-Atomen bedeuten.

5. Verwendung nach einem oder mehreren der Ansprüche 1 bis 4, **dadurch gekennzeichnet, dass** das Kation N-Methyl-4-(N',N'-dimethylamino)-pyridinium, N-Ethyl-4-(N',N'-dimethylamino)-pyridinium oder N-Butyl-4-(N',N'-dimethylamino)-pyridinium ist.

6. Verwendung nach einem oder mehreren der Ansprüche 1 bis 5, **dadurch gekennzeichnet, dass** das Anion der ionischen Flüssigkeit Cl⁻, Br⁻, I⁻ oder BF₄⁻ ist.

7. Verfahren für die Wiederfaltung, die Erhöhung der thermischen Stabilität und/oder die Verringerung der Proteinaggregation, bei dem die zu behandelnden Proteine mit einem flüssigen Medium in Kontakt gebracht werden, das mindestens eine ionische Flüssigkeit enthält, die ein Kation mit mindestens einer Elektronendonorregion und mindestens einer positiv geladenen elektrostatischen Region, die räumlich voneinander getrennt sind, und mit einer der folgenden allgemeinen Strukturen A oder B
[HetN]⁺-ED A
oder
[HetNED]⁺ B
enthält, wobei
HetN ein aromatisches, teilaromatisches oder nichtaromatisches heterocyclisches Ringsystem mit mindestens einem Stickstoffatom, das Teil des Ringsystems ist, bedeutet und
ED einen Elektronendonor bedeutet; **dadurch gekennzeichnet, dass** das Kation mit mindestens einer Elektronendonorregion und mindestens einer positiv geladenen elektrostatischen Region, die räumlich voneinander getrennt sind, aus der Gruppe 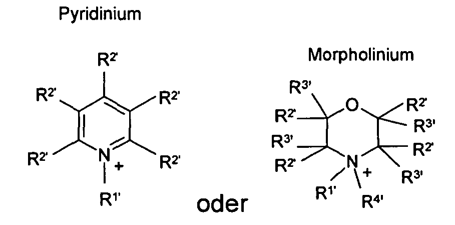 ausgewählt ist, wobei R^{1'}bis R^{4'} jeweils unabhängig voneinander bedeuten -H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂, -NO₂,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann, gesättigtes, teilweise oder vollständig ungesättigtes Heteroaryl, Heteroaryl-C₁-C₆-alkyl oder Aryl-C₁-C₆-alkyl,
wobei die Substituenten R^{1'}, R^{2'}, R^{3'} und/oder R^{4'} zusammen auch ein Ringsystem bilden können,
wobei ein oder mehrere Substituenten R^{1'} bis R^{4'} teilweise oder vollständig durch Halogene, insbesondere -F und/oder -Cl, oder -OH, -OR', -CN, -C(O)OH, -C(O)NR'₂, -SO₂NR'₂, -C(O)X, -SO₂OH, -SO₂X oder -NO₂, substituiert sein können, wobei aber R^{1'} und R^{4'} nicht gleichzeitig vollständig durch Halogene substituiert sein können, und wobei ein oder zwei nicht benachbarte Kohlenstoffatome der Substituenten R^{1'} bis R^{4'}, die nicht an das Heteroatom gebunden sind, ersetzt sein können durch Atome und/oder Atomgruppen ausgewählt aus -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- und -P(O)R'-, wobei R' = H, nicht, teilweise oder perfluoriertes C₁- bis C₆-Alkyl, C₃- bis C₇-Cycloalkyl oder unsubstituiertes oder substituiertes Phenyl und X = Halogen,
mit der Maßgabe, dass das Pyridiniumkation mindestens einen Substituenten R^{1'} bis R^{4'} pro Molekül, bei dem es sich um -CN, -OR', -NR'₂, -P(O)R'₂, -P(O)(OR')₂, -P(O)(NR'₂)₂, -C(O)R', -C(O)OR', -C(O)NR'₂, -SO₂NR'₂ oder -NO₂ handelt, und/oder mindestens einen Substituenten R^{1'} bis R^{4'} pro Molekül, der mit -OR', -CN, -C(O)OH, -C(O)NR'₂, -SO₂NR'₂, -C(O)X, -SO₂OH, -SO₂X oder -NO₂ substituiert ist oder in dem ein oder zwei nicht benachbarte Kohlenstoffatome, die nicht an das Heteroatom gebunden sind, ersetzt sind durch Atome und/oder Atomgruppen ausgewählt aus -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- und -P(O)R'-, aufweist.

8. Verfahren zur Extraktion von Proteinen aus Einschlusskörpern, indem man
a) die Proteine aus den Einschlusskörpern mit denaturierenden oder chaotropen Mitteln herauslöst,
b) die in Schritt a) herausgelösten Proteine wieder faltet, indem man sie mit einem flüssigen Medium in Kontakt bringt, das mindestens eine ionische Flüssigkeit enthält, die ein Kation mit mindestens einer Elektronendonorregion und mindestens einer positiv geladenen elektrostatischen Region, die räumlich voneinander getrennt sind, und mit einer der folgenden allgemeinen Strukturen A oder B
[HetN]⁺-ED A
oder
[HetNED]⁺ B
enthält, wobei
HetN ein aromatisches, teilaromatisches oder nichtaromatisches heterocyclisches Ringsystem mit mindestens einem Stickstoffatom, das Teil des Ringsystems ist, bedeutet und
ED ein Elektronendonor ist; **dadurch gekennzeichnet, dass** das Kation mit mindestens einer Elektronendonorregion und mindestens einer positiv geladenen elektrostatischen Region, die räumlich voneinander getrennt sind, aus der Gruppe 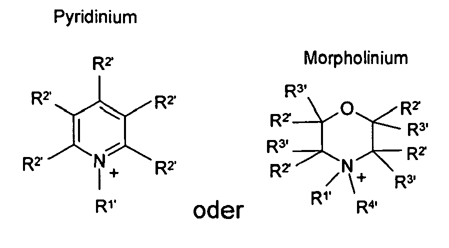 ausgewählt ist, wobei R^{1'} bis R^{4'} jeweils unabhängig voneinander bedeuten -H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂, -NO₂,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann, gesättigtes, teilweise oder vollständig ungesättigtes Heteroaryl, Heteroaryl-C₁-C₆-alkyl oder Aryl-C₁-C₆-alkyl,
wobei die Substituenten R^{1'}, R^{2'}, R^{3'} und/oder R^{4'} zusammen auch ein Ringsystem bilden können,
wobei ein oder mehrere Substituenten R^{1'} bis R^{4'} teilweise oder vollständig durch Halogene, insbesondere -F und/oder -Cl, oder -OH, -OR', -CN, -C(O)OH, -C(O)NR'₂, -SO₂NR'₂, -C(O)X, -SO₂OH, -SO₂X oder -NO₂, substituiert sein können, wobei aber R^{1'} und R^{4'} nicht gleichzeitig vollständig durch Halogene substituiert sein können, und wobei ein oder zwei nicht benachbarte Kohlenstoffatome der Substituenten R^{1'} bis R^{4'}, die nicht an das Heteroatom gebunden sind, ersetzt sein können durch Atome und/oder Atomgruppen ausgewählt aus -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- und -P(O)R'-, wobei R' = H, nicht, teilweise oder perfluoriertes C₁- bis C₆-Alkyl, C₃- bis C₇-Cycloalkyl oder unsubstituiertes oder substituiertes Phenyl und X = Halogen,
mit der Maßgabe, dass das Pyridiniumkation mindestens einen Substituenten R^{1'} bis R^{4'} pro Molekül, bei dem es sich um -CN, -OR', -NR'₂, -P(O)R'₂, -P(O)(OR')₂, -P(O)(NR'₂)₂, -C(O)R', -C(O)OR', -C(O)NR'₂, -SO₂NR'₂ oder -NO₂ handelt, und/oder mindestens einen Substituenten R^{1'} bis R^{4'} pro Molekül, der mit -OR', -CN, -C(O)OH, -C(O)NR'₂, -SO₂NR'₂, -C(O)X, -SO₂OH, -SO₂X oder -NO₂ substituiert ist oder in dem ein oder zwei nicht benachbarte Kohlenstoffatome, die nicht an das Heteroatom gebunden sind, ersetzt sind durch Atome und/oder Atomgruppen ausgewählt aus -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- und -P(O)R'-, aufweist.

## Revendications

1. Utilisation de liquides ioniques comprenant un cation ayant au moins une région donneuse d'électrons et au moins une région électrostatique chargée positivement qui sont spatialement distinctes l'une de l'autre, et répondant à l'une des structures générales suivantes A ou B
[HétN]⁺-ED A
ou
[HétNED]⁺ B,
HétN étant un noyau hétérocyclique aromatique, partiellement aromatique ou non aromatique, au moins un atome d'azote faisant partie du noyau et
ED étant un donneur d'électrons pour le repliement, pour l'augmentation de la stabilité thermique et/ou pour la diminution de l'agrégation des protéines ;
**caractérisée en ce que** le cation ayant au moins une région donneuse d'électrons et au moins une région électrostatique chargée positivement qui sont spatialement distinctes l'une de l'autre, est choisi dans le groupe constitué par 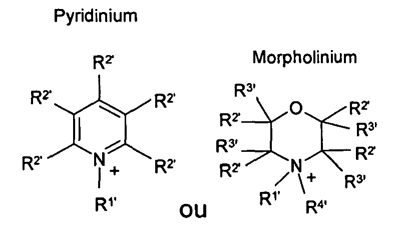 où R^{1'} à R^{4'} désignent chacun, indépendamment l'un de l'autre, -H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂, -NO₂,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
hétéroaryle, hétéroaryl-C₁-C₆-alkyle ou aryl-C₁-C₆-alkyle saturé, partiellement ou totalement insaturé,
où les substituants R^{1'}, R^{2'}, R^{3'} et/ou R^{4'} peuvent également former ensemble un noyau,
où un ou plusieurs substituants R^{1'} à R^{4'} peuvent être partiellement ou totalement substitués par des halogènes, en particulier -F et/ou -Cl, ou -OH, -OR', -CN, -C(O)OH, -C(O)NR'₂, -SO₂NR'₂, -C(O)X, -SO₂OH, -SO₂X ou -NO₂, mais où R^{1'} et R^{4'} ne peuvent être simultanément totalement substitués par des halogènes, et où un ou deux atomes de carbone non adjacents des substituants R^{1'} à R^{4'} qui ne sont pas liés à l'hétéroatome peuvent être remplacés par des atomes et/ou des groupes d'atomes choisis parmi -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- et -P(O)R'-, où R' = H, C₁- à C₆-alkyle, C₃- à C₇-cycloalkyle non fluoré, partiellement fluoré ou perfluoré, ou phényle non substitué ou substitué, et X = halogène,
à condition que le cation pyridinium ait au moins un substituant R^{1'} à R^{4'} par molécule qui soit -CN, -OR', -NR'₂, -P(O)R'₂, -P(O)(OR')₂, -P(O)(NR'₂)₂, -C(O)R', -C(O)OR', -C(O)NR'₂, -SO₂NR'₂ ou -NO₂ et/ou au moins un substituant R^{1'} à R^{4'} par molécule qui soit substitué par -OR', -CN, -C(O)OH, -C(O)NR'₂, -SO₂NR'₂, -C(O)X, -SO₂OH, -SO₂X ou -NO₂ ou où un ou deux atomes de carbone non adjacents qui ne sont pas liés à l'hétéroatome sont remplacés par des atomes et/ou des groupes d'atomes choisis parmi -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- et -P(O)R'- .

2. Utilisation selon la revendication 1, **caractérisée en ce que** les substituants R' sont, indépendamment l'un de l'autre, méthyle, éthyle, isopropyle, propyle, butyle, sec-butyle, tertio-butyle, pentyle ou hexyle.

3. Utilisation selon les revendications 1 et 2, **caractérisée en ce qu'**un et seulement un substituant R^{1'} à R^{4'} par molécule est -CN, -OR', -NR'₂, -P(O)R'₂, -P(O)(OR')₂, -P(O)(NR'₂)₂, -C(O)R', -C(O)OR', -C(O)NR'₂, - SO₂NR'₂ ou -NO₂.

4. Utilisation selon l'une ou plusieurs parmi les revendications 1 à 3, **caractérisée en ce qu'**un et seulement un substituant R^{1'} à R^{4'} par molécule est -CN, -OR', -NR'₂, -P(O)R'₂, -P(O)(OR')₂, -P(O)(NR'₂)₂, -C(O)R', -C(O)OR', -C(O)NR'₂, -SO₂NR'₂ ou -NO₂ et les autres substituants R^{1'} à R^{4'} désignent, indépendamment l'un de l'autre, -H ou alkyle à chaîne linéaire ou ramifiée ayant 1-20 atomes de C.

5. Utilisation selon l'une ou plusieurs parmi les revendications 1 à 4, **caractérisée en ce que** le cation est le N-méthyl-4-(N',N'-diméthylamino)-pyridinium, le N-éthyl-4-(N',N'-diméthylamino)-pyridinium ou le N-butyl-4-(N',N'-diméthylamino)-pyridinium.

6. Utilisation selon l'une ou plusieurs parmi les revendications 1 à 5, **caractérisée en ce que** l'anion du liquide ionique est Cl⁻, Br⁻ , I⁻ ou BF₄⁻.

7. Méthode de repliement, d'augmentation de la stabilité thermique et/ou de diminution de l'agrégation de protéines, où les protéines à traiter sont mises en contact avec un milieu liquide comprenant au moins un liquide ionique qui comprend un cation ayant au moins une région donneuse d'électrons et au moins une région électrostatique chargée positivement qui sont spatialement distinctes l'une de l'autre, et répondant à l'une des structures générales suivantes A ou B
[HétN]⁺-ED A
ou
[HétNED]⁺ B,
HétN étant un noyau hétérocyclique aromatique, partiellement aromatique ou non aromatique, au moins un atome d'azote faisant partie du noyau et
ED étant un donneur d'électrons ; **caractérisée en ce que** le cation ayant au moins une région donneuse d'électrons et au moins une région électrostatique chargée positivement qui sont spatialement distinctes l'une de l'autre, est choisi dans le groupe constitué par 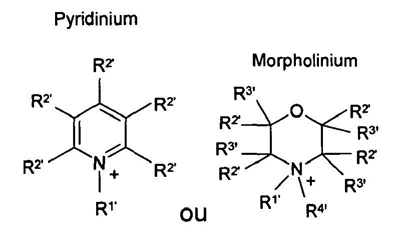 où R^{1'} à R^{4'} désignent chacun, indépendamment l'un de l'autre, -H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂, -NO₂,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
hétéroaryle, hétéroaryl-C₁-C₆-alkyle ou aryl-C₁-C₆-alkyle saturé, partiellement ou totalement insaturé,
où les substituants R^{1'}, R^{2'}, R^{3'} et/ou R^{4'} peuvent également former ensemble un noyau,
où un ou plusieurs substituants R^{1'} à R^{4'} peuvent être partiellement ou totalement substitués par des halogènes, en particulier -F et/ou -Cl, ou -OH, -OR', -CN, -C(O)OH, -C(O)NR'₂, -SO₂NR'₂, -C(O)X, -SO₂OH, -SO₂X ou -NO₂, mais où R^{1'} et R^{4'} ne peuvent être simultanément totalement substitués par des halogènes, et où un ou deux atomes de carbone non adjacents des substituants R^{1'} à R^{4'}qui ne sont pas liés à l'hétéroatome peuvent être remplacés par des atomes et/ou des groupes d'atomes choisis parmi -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- et -P(O)R'-, où R' = H, C₁- à C₆-alkyle, C₃- à C₇-cycloalkyle non fluoré, partiellement fluoré ou perfluoré, ou phényle non substitué ou substitué, et X = halogène,
à condition que le cation pyridinium ait au moins un substituant R^{1'} à R^{4'} par molécule qui soit -CN, -OR', -NR'₂, -P(O)R'₂, -P(O)(OR')₂, -P(O)(NR'₂)₂, -C(O)R', -C(O)OR', -C(O)NR'₂, -SO₂NR'₂ ou -NO₂ et/ou au moins un substituant R^{1'} à R^{4'} par molécule qui soit substitué par -OR', -CN, -C(O)OH, -C(O)NR'₂, -SO₂NR'₂, -C(O)X, -SO₂OH, -SO₂X ou -NO₂ ou où un ou deux atomes de carbone non adjacents qui ne sont pas liés à l'hétéroatome sont remplacés par des atomes et/ou des groupes d'atomes choisis parmi -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- et -P(O)R'-.

8. Méthode d'extraction de protéines à partir de corps d'inclusion, par
a) la solubilisation des protéines à partir des corps d'inclusion à l'aide d'agents dénaturants ou d'agents chaotropiques,
b) le repliement des protéines solubilisées dans l'étape a) par leur mise en contact avec un milieu liquide comprenant au moins un liquide ionique qui comprend un cation ayant au moins une région donneuse d'électrons et au moins une région électrostatique chargée positivement qui sont spatialement distinctes l'une de l'autre, et répondant à l'une des structures générales suivantes A ou B
[HétN]⁺-ED A
ou
[HétN ED]⁺ B,
HétN étant un noyau hétérocyclique aromatique, partiellement aromatique ou non aromatique, au moins un atome d'azote faisant partie du noyau et
ED étant un donneur d'électrons ; **caractérisée en ce que** le cation ayant au moins une région donneuse d'électrons et au moins une région électrostatique chargée positivement qui sont spatialement distinctes l'une de l'autre, est choisi dans le groupe constitué par 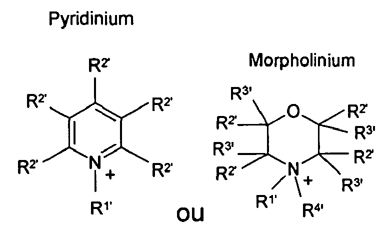 où R^{1'} à R^{4'} désignent chacun, indépendamment l'un de l'autre, -H, -CN, -OR', -NR'₂, -P(O)R'₂, -P(O)(OR')₂, -P(O)(NR'₂)₂, -C(O)R', -C(O)OR', -C(O)NR'₂, -SO₂NR'₂, -NO₂,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
hétéroaryle, hétéroaryl-C₁-C₆-alkyle ou aryl-C₁-C₆-alkyle saturé, partiellement ou totalement insaturé,
où les substituants R^{1'}, R^{2'}, R^{3'} et/ou R^{4'} peuvent également former ensemble un noyau,
où un ou plusieurs substituants R^{1'} à R^{4'} peuvent être partiellement ou totalement substitués par des halogènes, en particulier -F et/ou -Cl, ou -OH, -OR', -CN, -C(O)OH, -C(O)NR'₂, -SO₂NR'₂, -C(O)X, -SO₂OH, -SO₂X ou -NO₂, mais où R^{1'} et R^{4'} ne peuvent être simultanément totalement substitués par des halogènes, et où un ou deux atomes de carbone non adjacents des substituants R^{1'} à R^{4'} qui ne sont pas liés à l'hétéroatome peuvent être remplacés par des atomes et/ou des groupes d'atomes choisis parmi -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- et -P(O)R'-, où R' = H, C₁- à C₆-alkyle, C₃- à C₇-cycloalkyle non fluoré, partiellement fluoré ou perfluoré, ou phényle non substitué ou substitué, et X = halogène,
à condition que le cation pyridinium ait au moins un substituant R^{1'} à R^{4'} par molécule qui soit -CN, -OR', -NR'₂, -P(O)R'₂, -P(O)(OR')₂, -P(O)(NR'₂)₂, -C(O)R', -C(O)OR', -C(O)NR'₂, -SO₂NR'₂ ou -NO₂ et/ou au moins un substituant R^{1'} à R^{4'} par molécule qui soit substitué par -OR', -CN, -C(O)OH, -C(O)NR'₂, -SO₂NR'₂, -C(O)X, -SO₂OH, -SO₂X ou -NO₂ ou où un ou deux atomes de carbone non adjacents qui ne sont pas liés à l'hétéroatome sont remplacés par des atomes et/ou des groupes d'atomes choisis parmi -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- et -P(O)R'-.
